# EUROPEAN PATENT APPLICATION

(11) **EP 1 080 725 A1**
(43) Date of publication of application: **07.03.2001**
(21) Application number: 99202724.3
(22) Date of filing: 23.08.1999
(51) Int. Cl.: A61K 31/495

(54) **Use of fluoroquinolones for treating atherosclerosis**

(71) Applicant: Universiteit Utrecht, 3584 CS Utrecht (NL)
(72) Inventor: Herings, R.M.C. c/o Universiteit Utrecht, 3854 CS Utrecht (NL)
(74) Representative: Ottevangers, Sietse Ulbe

(57) **Abstract**

The present invention relates to the use of a fluoroquinolone in the preparation of a medicament for the treatment of atherosclerosis. In addition, the present invention is directed to a method of treating atherosclerosis, wherein a fluoroquinolone is administered to a human or animal.

## Description

The present invention is directed to the treatment of atherosclerosis and with atherosclerosis associated diseases, and more in particular relates to the use of certain known antibiotics in this treatment.

In a recent article in JAMA (1999), 281: 427-431, Meier et al. have described that the use of certain antibiotics, and in particular tetracyclines and quinolones, protect against the development of myocardial infarction. This finding seemed to corroborate previous evidence on a higher incidence of myocardial infarction among persons with antibodies against *Chlamydia pneumoniae* (Danesh *et al*., Lancet (1997), 350: 430-436); the results of two small randomized trials on antibiotic use and recurrence of myocardial infarction (Gupta S. *et al.*, Circulation (1997) 96: 404-407; Gurfinkel E. et al., Lancet (1997); 350: 404-407) ; evidence on the presence of *C. pneumoniae* in atherosclerotic plaques (Gupta S. and Camm A.J., BMJ (1997); 314: 1778); and on a molecular mimicry between *C. pneumoniae* and antigens in mouse heart-muscle cells (Bachmaier K. *et al.*, Science (1999); 283: 1335-1339).

In an attempt to confirm the negative association between antibiotic use and the development of myocardial infarction, the present inventors have investigated a large pharmaco-epidemiologic data-base in the Netherlands. The present inventors used a case-control design, in which persons were searched for with a first hospitalisation for acute myocardial infarction, while they specifically focussed on antibiotics against *C. pneumoniae*, such as antibiotics of the classes of the tetracyclines, the macrolides and the (fluoro)quinolones.

It has now surprisingly been found that fluoroquinolones lead to an irreversible or at least a direct inhibition of atherosclerosis. The inhibition is irreversible or direct in the sense that it is not associated with the mere killing or inactivation of *C. pneumoniae,* because other known antibiotics against this microorganism do not have the same effect.

Hence, the present invention relates to the use of a fluoroquinolone in the preparation of a medicament for the treatment of atherosclerosis. In the present description and the appended claims, the term "atherosclerosis" also encompasses diseases that are directly related to atherosclerosis.

In a second aspect, the present invention relates to a method for treating atherosclerosis wherein a fluoroquinolone is administered to a human or animal.

The present invention encompasses the use of fluoroquinolones in the therapy, but also in the profylaxis of artherosclerosis. In preferred embodiments the fluoroquinolones are prepared or used in formulations suitable for oral and parenteral use.

The fluoroquinolones are a group of chemical compounds having antibacterial activity. More in particular, these compounds have the formula: 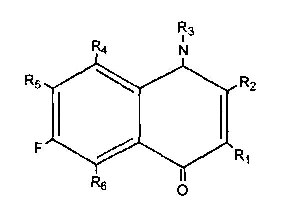 wherein R₁ represents a -COOH or -COOR, wherein R is a C₁₋₆ linear, branched or cyclic alkyl or alkylene group, which group may be substituted with a halogen, a hydroxy or amino group, but preferably is a -COOH; wherein R₂ and R₆ represent a hydrogen, hydroxy, amino or a C₁₋₄ linear, branched or cyclic alkyl or alkylene group, which group may be substituted with a halogen, a hydroxy or amino group, but preferably are hydrogen atoms; wherein R₃ represents hydrogen, hydroxy, amino or a C₁₋₆ linear, branched or cyclic alkyl or alkylene group, which group may be substituted with a halogen, a hydroxy or amino group, preferably a C₁₋₄ linear, branched or cyclic alkyl group, more preferably an ethyl or cyclopropyl group; wherein R₄ represents a hydrogen, hydroxy, amino or a C₁₋₄ linear, branched or cyclic alkyl or alkylene group, which group may be substituted with a halogen, a hydroxy or amino group, but preferably is a hydrogen atom; or wherein R₃ and R₄ together form a moiety -CHX-CH₂-O- group, wherein X is a C₁₋₄ alkyl group; and wherein R₅ represents a mono-substituted, di-substituted or cyclic substituted amino group which may contain a heteroatom, such as an oxygen or nitrogen atom, and may have substituents such as hydroxy, an alkyl having 1-6 carbon atoms, amino or halogen, but preferably is a piperazinyl group.

Preferably, the fluoroquinolones to be used in accordance with the present invention have the formula 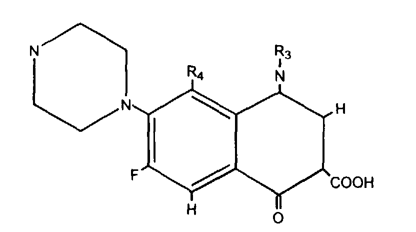 wherein R₃ and R₄ have the above mentioned meanings.

These fluoroquinolone antibiotics and their preparation methods are well-known in the art and described in, e.g., DE-OS-31 42 854, US-A-4,670,444, US-A-4,146,719, US-A-4,292,317 and EP-A-0 047 005.

In a preferred embodiment, the fluoroquinolone used is ciprofloxacin. However, also norfloxacine, ofloxacine and trovafloxacine can suitably be used, as well as salts, such as acid addition salts, e.g., the lactates thereof, and solvates of these compounds.

According to the present invention, it has been found that - although there are hypotheses in the prior art that there is a relation between infections caused by microorganisms and in particular by *C. pneumoniae* and the occurrence of atherosclerosis and diseases associated therewith - antibiotics against these microorganisms do in general not have a protective or inhibitory effect on these diseases, but that high dosages of fluoroquinolones do have an effect. This means that fluoroquinolones directly work on the atherosclerotic plaque formation. Without wishing to be bound to some hypothesis, it is assumed that fluoroquinolones either stop atherosclerotic plaque formation and/or dissolve plaque from the vessel walls.

The fluoroquinolones are in accordance with the present invention used in rather high doses and in courses for at least 5 and preferably at least 6 days. For oral applications, e.g., ciprofloxacine (e.g., Ciproxin® suspensions or tablets, Raxar® tablets, or Tavanic® tablets) is used in daily doses of at least 800 and preferably at least 1000 mg; norfloxacine (e.g., Noroxin® tablets) is used in daily doses of at least 700 mg and preferably at least 800 mg; ofloxacine (e.g., Tarivid® tablets) is used in daily doses of at least 300 mg and preferably at least 400 mg; and trovafloxacine (e.g., Trovan® tablets) is used in daily doses of at least 150 mg and preferably at least 200 mg. The amounts to be used daily depend on the pharmacon used, but are generally less than 2000 mg, preferably less than 1500 mg.

For parenteral applications, e.g., ciprofloxacine (e.g., Ciproxin® infusion liquids, or Tavanic® infusion liquids) is used in daily doses of at least 400 and preferably at least 500 mg; ofloxacine (e.g., Tarivid® infusion liquids) is used in daily doses of at least 300 mg and preferably at least 400 mg; and trovafloxacine (e.g., Trovan® infusion liquids) is used in daily doses of at least 150 mg and preferably at least 200 mg. The amounts to be used daily depend on the pharmacon used, but are generally less than 1000 mg, preferably less than 800 mg.

More in detail, the invention is described hereinbelow.

The present inventors have obtained the information needed to confirm the results of the investigations of Meier et al. from the PHARMO system, a population-based automated pharmacy data system, which includes information of hospital admissions and drug-dispensing records for all 450.000, residents of 8 Dutch cities. This PHARMO system is described, e.g., in Herings et al., J. Epidemiology and Community Health (1992); 46: 136-140 and by Herings and Leufkens, Community Pharmacy Networks in Pharmacoepidemiology, in: Hartzema et al., Pharmaco-epidemiology: An introduction, 2^{nd} edition, CincinnatiL Harcey, Whitney Books (1998); 405-420. The drug-dispensing records in this system were obtained from pharmacy files, are virtually complete, and are linked nation-wide to the patient's hospital discharge records with a linkage sensitivity and specificity of more than 95%. The system was established in 1989 and includes data for some cities back to 1986; since 1990 it has been updated for 6 cities (N=300.000) and from 1993 onwards for 8 cities (N=450.000). All available data from 1986 to 1995 have been used.

All persons having an age of 35-75 years who were firstly hospitalised for a primary diagnosis of myocardial infarction (ICD code 410; International Classification of Diseases; World Health Organization, Geneva) with a registration period of at least 3 years, were identified. For each of these patients the date of first entry into the PHARMO roster files was assessed.

In the same database up to four controls were selected. More in particular were controls matched on dispensing pharmacy, sex, year of birth, and eligibility in the database during a similar time and calendar period as the patients (derived from prescription and/or hospitalization dates).

Subsequently, the following groups were excluded:
a) all patients and controls with a known medical history indicating a secondary diagnosis of old myocardial infarction (ICD 412), cardiac aneurysm (ICD 414.1), prolonged angina pectoris, other coronary syndromes that are manifestations of atherosclerosis (ICD codes 411, 413, 414.0, 414.8, 414.9);
b) persons with a hospitalization for hypertension (ICD 401-405), ischaemic and other forms of heart disease (ICD 410-414) or cerebrovascular accidents (ICD 430-438); and
c) persons that were ever treated with a drug labeled for treatment of any cardiovascular disease or diabetes (e.g., angiotensine-converting-enzyme (ACE)-inhibitors, calcium channel (CA)-antagonists, low and high ceiling diuretics, anti-lipaemics, beta-blockers, other cardiac drugs, insulin and oral antidiabetics).

To study the comparability of patients and controls the Chronic Disease Scores (CDS) for each patient and control were assessed according to the method described by von Korff *et al.* in J. Clin. Epidemiol. (1992); 45 (2): 197-203. The measurement was based on the complete available history of the patients and controls. The outcome of the CDS score ranges from 0 (no chronic disease) to *n* (number of chronic diseases).

As patients and controls were both free from any hospitalisation or drugs use for treatment of any cardiovascular disease, the CDS score did therefore not include cardiovascular disease.

The exposure to antibiotics was restricted to the calendar time window, the time period wherein exposure was ascertained, prior to the index date; in the present case this time window was the total history of the case patient. Antibiotic use was classified into 7 classes: tetracyclines, macrolides (erythromycin and others), sulfonamides, (fluoro)quinolones, penicillins, cephalosporins, and the group of combinations of these antibiotics. The initial analyses referred to the exclusive use of a particular class of antibiotic (patients were only allowed the use of one class of antibiotics in the exposure time window, otherwise their use was coded as "Other/Switchers"), like in the publication by Meier *et al*. mentioned above. To study a dose-response relationship and since only higher dosages might be sufficient to eradicate *C. pneumoniae,* high doses were defined as courses longer than 6 days with a daily dose for fluoroquinolones and macrolides (excluding erythromycin) exceeding the recommended daily doses for these antibiotics (for fluoroquinolones > 1000 mg ciprofloxacin per day; for macrolides > 125 mg clarithromycin per day). For erythromycin and the tetracyclines a high dose was defined as a dose exceeding twice the recommended daily dose (> 600 mg erythromycin per day, and for tetracyclines > 200mg doxycyclin per day).

If patients were treated with several courses in subsequent years, they were classified as having received a high dose based on at least a single high dose course. The relationship with the frequency of courses of the different antibiotics were also studied. Finally, the differences between a single course versus more than one course, for ever use of at least five days, were taken into consideration. The analysis was carried out by two-by-two tables and conditional logistic regression analysis using EGRET®.

628 patients and 1615 controls that met the inclusion criteria of the study were identified (variable patient-to-control ratio: 121 patients matched 1:1, 176 matched 1:2, 182 matched 1:3, 159 matched 1:4).

All cases had a first-ever hospitalisation myocardial infarction (Table 1) and patients and controls were free from any cardiovascular treatment and hospitalisation prior to their index date. Patient and control groups were not different with respect to the number of hospitalisations, treatment for respiratory complaints or presence of chronic diseases. The median age was 57 years (25-75 percentile: 49-65); almost 80% was male.

**Table 1:**

| Baseline characteristics for patients with a first hospitalization for acute myocardial infarction and matched controls | | | |
|---|---|---|---|
| | Patients, No. (%) (n=628) | Controls, No. (%) (n=1615) | Matched Odds Ratio (95%CI) |
| Age, years | | | |
| 35-49 | 160 (25.5) | 525 (32.5) | - |
| 50-64 | 301 (47.9) | 773 (47.9) | - |
| 65-75 | 167 (26.6) | 317 (19.6) | - |
| Gender | | | |
| Male | 517 (80.7) | 1341 (83.0) | - |
| Female | 122 (19.3) | 274 (17.0) | - |
| History (py)* | | | |
| 2-5 years | 363 (57.8) | 923 (57.6) | - |
| > 5 years | 265 (42.2) | 692 (42.4) | - |
| Median | 4.5 (3.1- | 4.5 (3.1-6.6) | - |
| | 6.6) | | |
| CDS-score* | | | |
| 0 | 389 (61.9) | 1056 (64.6) | 1.0 (ref.) |
| 1 | 114 (18.2) | 258(16.0) | 1.2 |
| | | | (0.9-1.5) |
| >1 | 125 (19.9) | 301 (18.6) | 1.1 |
| | | | (0.8-1.3) |
| Hospitalisations | 95 (15.1) | 215 (13.3) | 1.0 |
| | | | (0.9-1.2) |
| Respiratory | 66 (10.5) | 151 (9.4) | 1.0 |
| Medication | | | (0.8-1.4) |

| | | | |
|---|---|---|---|
| *: py = person-year; a history of x person year meaning a time window of at least x years. | | | |

Table 2 gives the analysis for "exclusive use" of particular antibiotics - the category of "Other/Switcher" contains all persons who used more than one type of antibiotic in the exposure window. Relative risks less than unity were found for tetracyclines, macrolides and (fluoro)quinolones - but all with small numbers and wide confidence intervals due to the "exclusive use" restriction.

**Table 2:**

| Risk of acute myocardial infarction among patients with history of exclusive use of a single type of antibiotics. | | | |
|---|---|---|---|
| Antibiotics | Patients (n=628) | Controls (n=1615) | Matched Odds Ratio (95%CI) |
| No. | 325 | 870 | 1.0 (ref.) |
| Tetracyclines | 44 | 128 | 0.89 (0.62-1.30) |
| Macrolides | 2 | 8 | 0.62 (0.12-3.10) |
| Sulfonamides | 17 | 28 | 1.65 (0.87-3.12) |
| Quinolones* | 3 | 10 | 0.77 (0.20-2.89) |
| Penicillins | 59 | 151 | 1.02 (0.73-1.44) |
| Cephalosporins | 0 | 4 | - |
| Other/ | 178 | 416 | 1.09 (0.87-1.38) |
| Switchers | | | |

| | | | |
|---|---|---|---|
| * Fluoroquinolones 3/6. | | | |

Table 3 presents "ever use" of a particular class of antibiotics during the exposure window, subdivided in high and low dosages. Only for high dosages of fluoroquinolones a consistent negative effect remains. Table 4 presents a different way of looking at a possible "dose-response" by subdividing according to the number of courses. The same negative effect, be it with wider confidence intervals, was found for more than one course of fluoroquinolones.

**Table 3:**

| Table 3: Admission for myocardial infarction and ever-use of different antibiotics | | | |
|---|---|---|---|
| Group of antibiotics | Cases (n=628) | Controls (n=1615) | Matched Odds Ratio (95%CI) |
| Fluoroquinolones | | | |
| Low dose | 5 (0.8) | 14 (0.9) | 1.05 (0.35-3.15) |
| High dose | 5 (0.8) | 32 (2.0) | **0.34 (0.12-0.93)** |
| Quinolones | | | |
| Low dose | 1 (.2) | 3 (0.2) | 1.01 (0.09-10.8) |
| High dose | 4 (0.6) | 11 (0.7) | 0.81 (0.24-2.73) |
| Tetracyclines | | | |
| Low dose | 170 | 379 | 1.13 (0.90-1.42) |
| | (27.1) | (23.5) | |
| High dose | 6 (1.0) | 13 (0.8) | 0.95 (0.35-2.62) |
| Macrolides | | | |
| Low dose | 11 (1.8) | 25 (1.5) | 1.02 (0.48-2.14) |
| High dose | 13 (2.1) | 22 (1.4) | 1.58 (0.74-3.35) |
| Other | | | |
| Low dose | 131 | 290 | 1.22 (0.95-1.57) |
| | (20.9) | (18.0) | |
| High dose | 100 | 247 | 1.04 (0.78-1.38) |
| | (15.9) | (15,3) | |
| * see text for dose definitions | | | |

**Table 4:**

| Ever use of at least five days of 3 classes of antibiotics, according to number of courses | | | | |
|---|---|---|---|---|
| Group of antibiotics | | Cases (n-628) | Controls (n-1615) | Matched Odds Ratio (95% CI) |
| Tetracyclines | 1 | 98 | 243 | 1.04 |
| | course | | | (0.79-1.35) |
| | >1 course | 75 | 143 | 1.32 |
| | | | | (0.95-1.84) |
| Macrolides | 1 | 13 | 37 | 0.91 |
| | course | | | (0.47-1.75) |
| | >1 course | 9 | 8 | 2.50 |
| | | | | (0.92-6.48) |
| Fluoroquinolones | 1 | 10 | 23 | 1.00 |
| | course | | | (0.46-2.22) |
| | >1 course | 0 | 16 | **0.12** |
| | | | | **(0.02-0.94)*** |
| Quinolones | 1 | 4 | 10 | 0.71 |
| | course | | | (0.22-2.29) |
| | >1 course | - | - | |
| Other antibiotics | 1 | 132 | 200 | 1.21 |
| | course | | | (0.94-1.55) |
| | >1 course | 100 | 245 | 1.00 |
| | | | | (0.95-1.84) |

| | | | | |
|---|---|---|---|---|
| * estimated by substituting 1 case in the case group | | | | |

The above data leave the evidence uncertain that infections with microorganisms, e.g., with *C. pneumoniae,* are causally related to acute myocardial infarction. Only for fluoroquinolones, a protective effect was found for myocardial infarction. These data are not compatible with an inhibitory effect on microorganisms in general and *C. pneumoniae* in particular, since no effect of tetracyclines and macrolides were found, also not when these known antibiotics were given in high dosages and/or in multiple courses during sufficient time.

It was however found that fluoroquinolones showed a dose-response relationship. This means that there is a direct effect of fluoroquinolones on atherosclerosis, which direct effect cannot be identified for the other antibiotics used. Said in other words, fluoroquinolones do have an activity against atherosclerosis that is not, or at least not solely, based on the antibiotic activity of these compounds.

## Claims

1. Use of a fluoroquinolone in the preparation of a medicament for the treatment of atherosclerosis.

2. Use according to claim 1, wherein the fluoroquinolone is ciprofloxacin.

3. Method for treating atherosclerosis wherein a fluoroquinolone is administered to a human or animal.

4. The method according to claim 3, wherein the fluoroquinolone used is ciprofloxacin.

5. The method according to claim 3 or 4, wherein the daily dose is at least 1000 mg, and maximally 2500 mg.
